Europäisches Patentamt

**European Patent Office**

Office européen des brevets

(11) Publication number: **0 155 636**
**A2**

# (12) EUROPEAN PATENT APPLICATION

(21) Application number: **85102896.9**

(22) Date of filing: **13.03.85**

(51) Int. Cl.⁴: **A 41 B 13/02**

(30) Priority: **19.03.84 US 590802**

(43) Date of publication of application:
**25.09.85 Bulletin 85/39**

(84) Designated Contracting States:
**AT BE CH DE FR IT LI LU NL SE**

(71) Applicant: **KIMBERLY-CLARK CORPORATION**
**401 North Lake Street**
**Neenah Wisconsin 54956(US)**

(72) Inventor: **Pomplun, William S.**
**142 Villa Drive**
**Neeah Wisconsin(US)**

(72) Inventor: **Woon, Paul S.**
**1009 East Florida Avenue**
**Appleton Wisconsin(US)**

(72) Inventor: **Stevens, Robert A.**
**2335 North Meade Street**
**Appleton Wisconsin(US)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath**
**Maximilianstrasse 58**
**D-8000 München 22(DE)**

(54) **Disposable diaper with controlled elastic at the waist.**

(57) The invention relates to a diaper having a partial elastic waist along the front and rear waist of an elastic-legged disposable diaper. The elastic has a controlled tension and a controlled retraction that allows change of the waist of the diaper as the wearer shifts position without causing so much pressure on the skin that abrasion or reddening of the skin takes place. The waist elastic further is located outside the area of the fluff of the diaper. In a preferred form of diaper the elastic is in place in the center one-third to two-thirds of the front of the diaper and between about one-third and the total width and the edges of the tape attachment points on the back of the diaper. Further, the waist elastics are placed at least one-fourth inch away from the edge of the fluff and have a maximum possible elastic travel of between about 2 inches and 4 inches in an infant diaper.

FIG. 1

# DISPOSABLE DIAPER WITH CONTROLLED ELASTIC AT THE WAIST

## TECHNICAL FIELD

The present invention relates to improvements in disposable diapers. The invention particularly relates to a disposable diaper having a waist elastic that provides a controlled elasticity resulting in a preferred fit.

## BACKGROUND ART

The positioning of elastic members in the crotch area of contoured diapers is known and has been disclosed in U.S. Patent to Buell 3,860,003 and U.S. Patent to Woon, et al. 4,050,462. In these patents the elastic members are provided in the crotch area in order to provide improved sealing from leakage when the diaper is wet or soiled.

It is also known as illustrated in U.S. Patents 4,388,075 to Mesek et al. and U.S. 4,352,355 also to Mesek et al. that elastic members may be placed in the ends of the diaper that will become the front and back portion when the diaper is worn. Generally, these patents disclose the elastics as being in substantially the entire end portion. United Kingdom Patent Application GB 2,095,561 to Karami also discloses a diaper having elastic in the crotch portion as well as an elastic portion in the waist. It is disclosed in this patent that the interaction of the elastic in the waist and the crotch portion cooperate to improve fit and conformance on the body when used. However, it not clear as to the amount of tension that is desired in the elastic at the waist or to what extent the waist portions should be elasticized. Further, there is a problem with increased abrasion of the wearer's skin when the elastics in the waist and crotch interact to cause tighter fit to the body.

Therefore, there remains a need for a diaper with elasticized waist and legs that provides increased protection against leakage at the waist portion as well as a diaper that does not lead to redness or abrasion of the baby's skin at either the crotch or waist area.

## DISCLOSURE OF THE INVENTION

An object of this invention is to provide a diaper having improved fit.

A further additional object is to provide a diaper that provides a low rate of leakage without leaving red marks, irritating, or abrading the skin of the wearer.

These objects of the invention are accomplished by providing an elastic along the center part of the front and rear waist of a disposable elastic leg diaper. The waist elastic has a controlled tension and a controlled retraction that allows change of the waist of the diaper as the wearer shifts position without causing so much pressure on the skin that abrasion or reddening of the skin takes place. The elastic is only attached to part of the front and rear of the diaper as only limited travel and tension of the diaper is desired and because the outer edges of the front may fold in after fastening and abrade the wearer if the outer edges are elasticized. The waist elastic further is located outside the marginal edge of the fluff or absorbent pad of the diaper.

Preferably the invention elastic is placed in the center one-third to two-thirds of the front of the diaper and between about one-third of the width and the edges of the tape attachment points on the back of the diaper. Further, preferably the waist elastics are placed at least one-quarter inch away from the marginal edge of the fluff, have a maximum possible elastic travel of between about 2 inches and 4 inches in an infant diaper, and an elastic tension of between about 125 and 175 grams.

BRIEF DESCRIPTION OF THE DRAWINGS

FIG. 1 illustrates a plan view of a diaper in accordance with the invention.

FIG. 2 illustrates in cross-section the location of the elastic in the invention diaper.

FIG. 3 is a prospective view of a diaper in accordance with the invention.

FIG. 4 is a plan view of a diaper of the invention with only a rear waist elastic.

FIG. 5 is a plan view of a diaper of the invention in which the front elastic is split.

FIG. 6 is a prospective view of the diaper of FIG. 5.

MODES FOR CARRYING OUT THE INVENTION

The diaper of the invention provides numerous advantages over prior products. The diaper of the invention prevents leaking at the waist while not being so restrictive that the baby is marked or abraded during ordinary movement. Further, the diaper of the invention is easier to fasten as the partial elastic in the back does not prevent easy access by the person applying the diaper to the fastening tapes which are located on the back. Diapers with elastic across the entire back portion are difficult to control when diapering as the tabs cannot be reached as they turn under the baby's body. An advantage of the partial elastic in the

-3-

front is that when the baby moves the edges that are against the baby and behind the anchoring point of the tape on the front portion do not have a tendency to curl inward. If the elastic extends beyond the anchoring point of the tape on the front of the diaper the elastic will cause the diaper edges to curl and the curled edges can lead both to abrasion of the baby's skin and to increased leakage. Further, the curling of the edge of the diaper may expose the fastening tape that could abrade the baby's skin. These and other advantages of the invention will become apparent from the further description below as the description of the drawings.

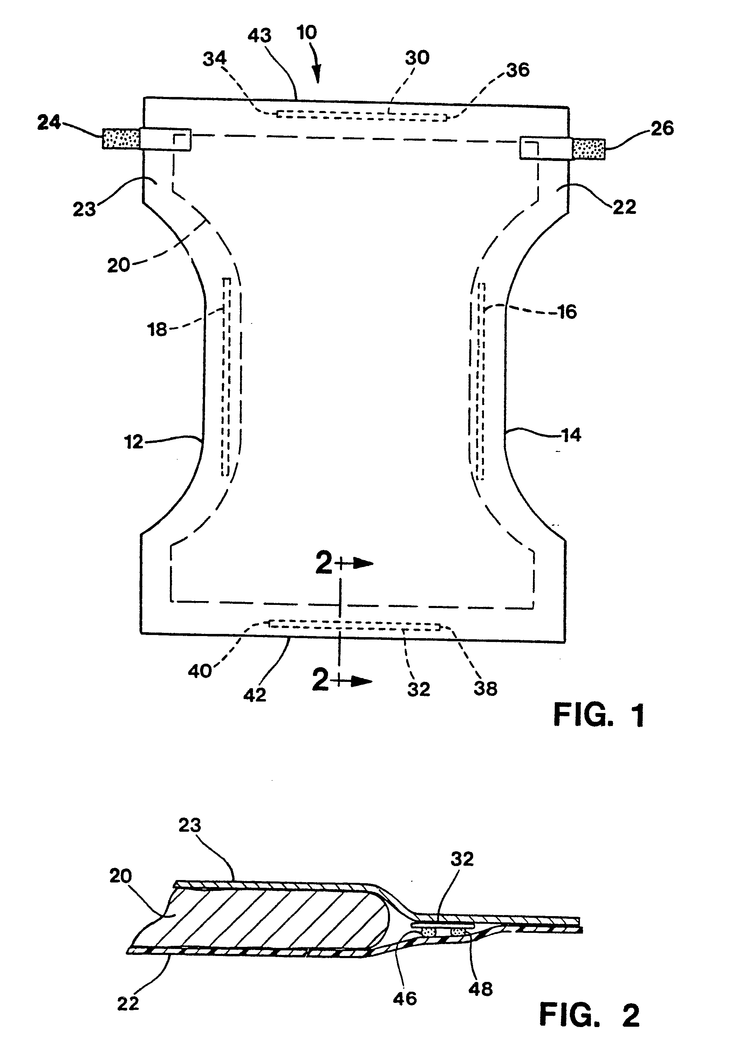

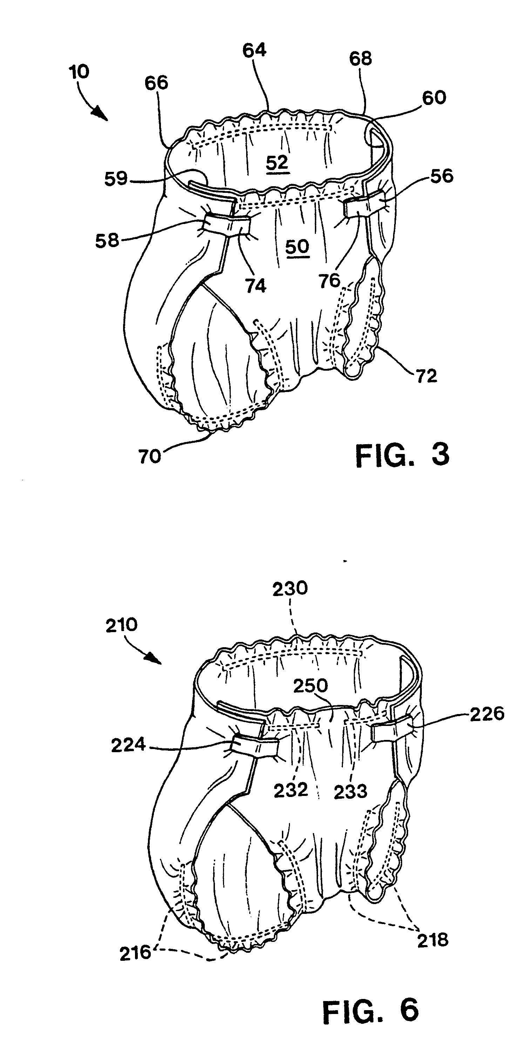

FIG. 1 is a plan view of a diaper 10 in accordance with the invention. The diaper in accordance with the invention has a contoured shape with leg cutouts 12 and 14. The diaper further has leg elastic pieces 16 and 18. It being noted that while these elastic pieces are shown as straight, it is also possible that they could be contoured. The diaper 10 further has an absorbent batt 20 that is smaller in size than the exterior impervious outer member of the diaper 22. The diaper is further provided with fastening tapes 24 and 26, which serve to fasten the diaper onto the wearer when in use. The diaper as illustrated in FIG. 1 is also provided with back elastic member 30 and front elastic 32. The back elastic has an effective length from 34 to 36 while the front elastic has an effective portion from 38 to 40. By "effective length," as utilized in this invention, it is meant the length of the elastic when fastened to the diaper in extended condition that serves to shorten the diaper by contraction of the elastic after fastening. The diaper is illustrated in extended condition. The elastics 30 and 32 are located between the margin of the absorbent batt 20 and the edges 42 and 43 of the diaper. The elastic is generally parallel to the edge of the diaper and parallel to the margin of the absorbent. "Travel" is the shortening

-4-

caused by contraction of the elastic and is the difference between the extended length and contracted length. Illustrated in FIG. 2 is a cross-section along section line 2-2 of FIG. 1. The front elastic member 32 is placed between the pervious inner liner 23 and the impervious outer covering 22. The elastic 32 is bonded to the polymer 22 by glue lines 46 and 48. While illustrated with two glue lines, it is within the invention to utilize evenly applied adhesive to adhere the elastic to the diaper or to utilize a self-adhesive elastic if desired. Further, it is possible that adhesive could be applied to both sides of the elastic 32 to adhere both the backing 22 and the liner 23 to the adhesive. It also may be possible to utilize heat sealing to adhere the elastic to the diaper backing 22 or the diaper backing 22 and the liner 23.

FIG. 3 is an illustration of a diaper in accordance with the invention, generally in the position it would be in when worn. The diaper of FIG. 3 has a front portion 50 and a rear portion 52. The diaper further is held in place by tapes 56 and 58 that are fastened to the front 50. The diaper has overlap portions 59 and 60 where the back 52 overlaps the front 50. These portions 59 and 60 should not be elasticized as they will wrinkle and fold over and possibly expose the fastening system when in use, causing abrasion to the wearer and also increasing the possibility of leakage. The back portion of the diaper is also elasticized at 64 but as illustrated, the elastic again is only in the center portions, leaving unelasticized edges 66 and 68. It is noted that there are also gathers formed by the elastics in the legs 70 and 72. One thing of particular note in accordance with the invention is that the anchoring points in the front of the diaper 74 and 76 where the tapes 56 and 58 are fastened determine the effective amount of elasticity in the front. The distance between the fastening points of these tapes provides the limits of travel for the front elastic. Elastic in excess of the expected anchoring areas 74 and 76 is wasted and actually is detrimental to performance of the diaper due to the tendency to fold over. Another thing of note in this invention is that the somewhat limited amount of elasticity in the waist of the diaper of the invention provides only limited travel of the waist and does not result in significant change in the functioning of the leg elastics. The leakage from leg elastics is generally substantially the same as if the waist was not elasticized. The primary benefit is that the waist elastic does aid in holding the diaper in place on the baby and preventing waist leakage. The performance of the leg elastic is substantially the same as the waist elastic and is not believed to substantially increase or decrease the tension at the legs or substantially increase the effectiveness of the leg elastic in preventing leakage.

As illustrated in FIG. 1, the waist elastics preferably are located outside of the area of the diaper occupied by the absorbent batt. Location there, parallel to the batt and the diaper edge, has been found to improve in conformance of the diaper to the body of the baby. The distance between the edge of the elastics 30 and 32 and the fluff 20 normally should not be less than one-eighth of an inch. A distance of at least about one-fourth inch has been found to be suitable. A preferred distance is greater than 1/2 inch as this optimizes retraction of the elastic without disrupting the integrity of the fluff and gives good utilization of the elastic shrinkage available. Generally a preferred range would be between one-half inch and the outer edge 42 or 43 of the diaper in order to give good results in prevention of leakage at the waist. The distance from the edge of the fluff 20 to the end of the diaper 42 or 43 is about one and one-quarter inch (1-1/4"). The waist elastic is preferably between the pervious liner and the impervious outer member as it improves the end seal and looks of the

-6-

diaper. However, the waist elastic could, if desired, be placed on the exterior of the impervious outer member.

As illustrated above, the control of the effective length of the elastic is a part of this invention. The front elastic is generally between about one-third and about two-thirds of the width of the front end of the diaper in the typical contoured diaper. The preferred amount of effective elastic length of the front elastic is between about six-tenths and about nine-twentieths of the total width as this range is generally the width between anchoring points of the tapes and is effective in preventing leakage from the front waist portion. The waist elastics are generally located in the center of the ends of the diaper. The back elastic may have a somewhat wider range of elasticity as there is no overlap of the back elastic. However, it should be noted that the back elastic length should be limited to the minimum effective amount as it will aid in application of the diaper to the wearer. Generally the upper limit for length of the back elastic is the length between the inside of the back tape attachment points. A lower limit of the length of the back elastic is about one-third of the total back width. The preferred amount of elastic in the back portion is between about one-third and about six-tenths, as this gives increased protection against leakage and provides enough width for travel of the elastic.

The amount of waist travel as used herein is defined as the amount of change in waist size that is possible for the diaper. Control of waist travel is important to formation of a diaper that does not cause reddening or abrasion of the wearer's skin while at the same time increasing protection against leakage when the wearer is lying down. Generally, when diapering a baby, the suitable amount of waist travel (combined travel of both the front and rear elastic) is between about 1 inch and about 7 inches. A preferred range

-7-

of waist travel is between about 2 inches and about 4 inches, as this is the amount which results in greatest increase in leakage protection from the waist when lying down. It is believed that the about 2 to about 4 inches range of travel is important as the typical baby changes that much in waist size between when in the sitting position and when lying down. Therefore, the diaper while it will not leak from the waist when the baby is sitting must be comfortable, especially when wet, so that the skin will not be reddened or abraded when the baby is sitting. The addition of the elastic at the waist further aids in the effectiveness of diapers in that differences in anchoring techniques of the person applying the diaper are minimized because the waist elastic will compensate somewhat for differences in anchoring points by different people doing the diapering.

The travel of the diaper in the front is particularly important. This is because the shape of the baby changes more in the front when the baby changes position and the front, therefore, is where failure of the diaper to prevent waist leakage is most likely. The travel of the elastic in the front of the diaper is suitably between about 1 and about 3 inches. The travel of the elastic in the back is generally suitably between about 1 and 4 inches. While generally described with elastic in both the front and the back of the diaper, it is possible that a diaper within the invention could have an elastic only in the back or only in the front. Such a diaper would be more effective than a diaper having no waist elastic and would be particularly effective if the elastic was placed in the front of the diaper and had a travel of between about 2 and about 3 inches. A diaper having elastic only in the back and having a travel between 2 and 4 inches also would be more effective than a nonelastic waist diaper. However, the diaper having elastic in both the back and the front is believed to most effective and further, has better appearance and is less likely to result in reddening or abrasion of the wearer's skin.

In accordance with the invention, it is also desirable that the tension caused by the expansion of the elastics on the diaper be controlled in order to prevent the marking of the baby's skin, causing redness or abrasion. Generally, the tension should be about the same in the front and back elastics of the diaper. The tension should be as low as will be sufficient to provide the required travel. The elastic tension may be any tension that provides a suitable fit of the diaper without skin reddening. Typical elastic tensions are between about 60 and 300 grams. A suitable tension for the diaper of the invention is between about 100 and about 200 grams. A preferred amount of tension is between about 125 and 175 grams tension to provide a functional diaper that will provide enough travel at the waist to decrease leakage while not reddening the skin of the baby. Therefore, it may be seen that the relationship of travel and tension of the elastic must be controlled such that the tension during the required travel as the baby changes position and waist size will not cause such an increase in tension that reddening of the skin will result.

The terms "travel" and "tension" have been discussed and specified to the above paragraphs. In the invention the travel is measured by the following procedures: A diaper is clamped at the side of one ear (i.e., where the tape is located on the back) and a 500 gram weight is hung downward, stretching the elastic of the end of the diaper in a straight downward manner. A template or ruler is used to mark a 5-inch segment or other length of the extended elastic length. The weight is removed, and the diaper is allowed to relax for two hours. Then the distance between the marked lines is again measured. The difference between the lines marked as extended length under the 500 gram load and the length between the lines after two hours is the travel. The travel of the measured portion is extrapolated to give travel for the total effective elastic length. The total travel for a diaper is the total of the measurements of the front and the back portion. Tension measurement, as used in description of this invention, is measured in the following way: The edge of the back or front of the diaper is again fastened in a clamp and a 500 gram weight is hung downward from the other ear of the diaper. A 5 inch or any suitable segment is marked onto the effective elastic portion while it is in extended condition. The weight is removed, and the diaper is allowed to relax for at least 2 hours. The diaper is then clamped such that one clamp is at one marked line. This clamp is placed into a spring scale such as an Ametek spring scale. A clamp is applied to the other marked line and then the elastic at the end of the diaper is extended to 90 percent of the original marked distance. In the instance of a 5 inch marked distance, the extension would be to 4½ inches. The diaper is held in this condition for 60 seconds and then the tension is read on the spring scale. Therefore, the tension measurement is a measurement of tension at 90 percent of the extension caused by a 500 gram weight.

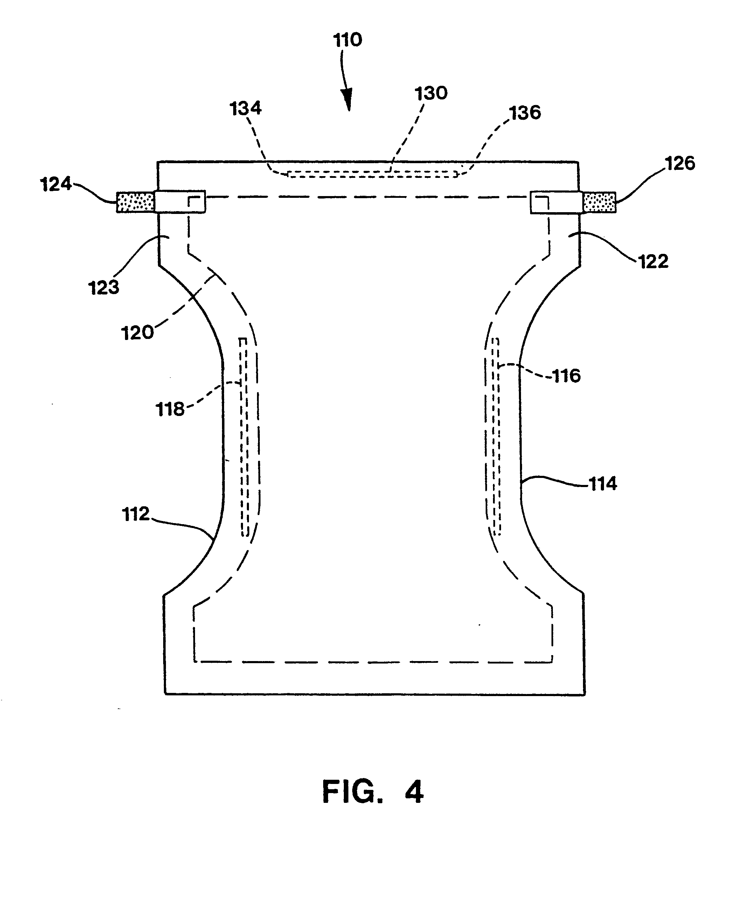

FIG. 4 illustrates another embodiment of the invention in which only the back portion of the diaper is elasticized. This structure while less effective than elastic in both front and rear does decrease the leakage, particularly in the back, when the baby is laying down. Further, as it is possible to elasticize a greater portion of the back, it is possible to place in excess of 2 inches of travel in just the back portion of the diaper. As illustrated in FIG. 4, diaper 110 has a rear waist elastic 130 having an effective length from 134 to 136. The diaper has tapes 124 and 126 outside the ends of the elastic. The fluff portion 120 does

-10-

not extend as far as the elastic, which is spaced well away from the fluff 120. The impervious outer layer 122 joins the generally coextensive pervious liner 123 at the outer edges. The diaper has leg cutouts 112 and 114 with leg elastic pieces 116 and 118. While illustrated in FIG. 4, with only a rear elastic it is also within the invention to only place an elastic in the front of the diaper.

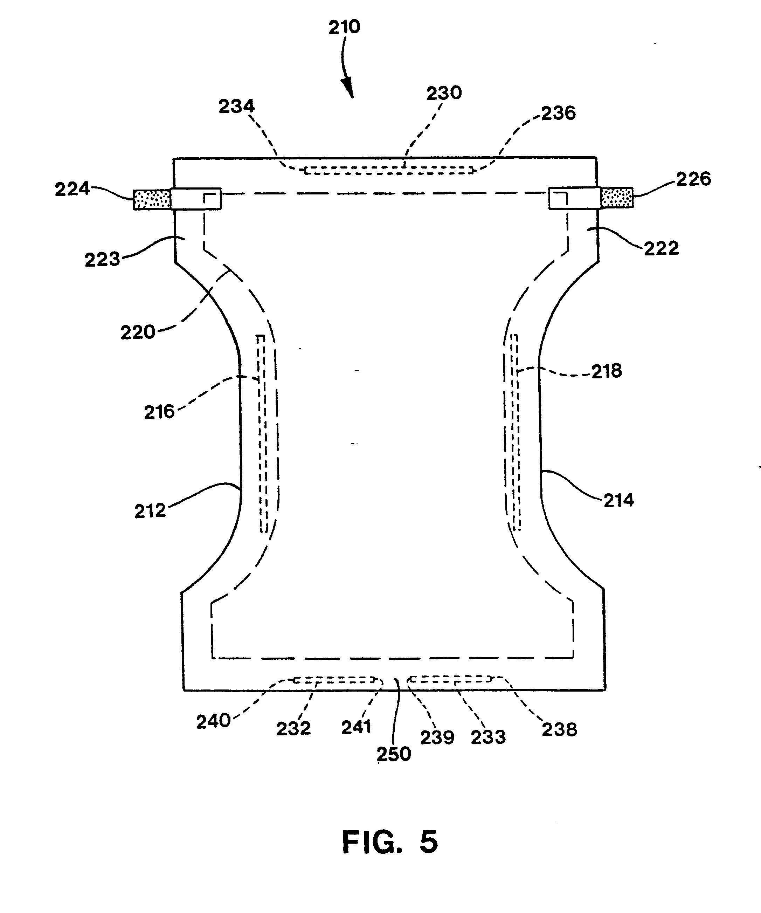

FIG. 5 and FIG. 6 illustrate another embodiment of the invention in which the front elastic has been divided into two pieces in order to avoid irritation of the navel. As there are many infants that have protruding navels and newborns have the umbilical that is healing, it may be important that the navel area not be irritated. This area being protruding and possibly healing is particularly sensitive to irritation, which would be accentuated by elastic. The diaper 210 has a back elastic 230 having an effective length between 234 and 236. The diaper has tapes 224 and 226 that are well outside the ends of the elastic. The diaper is composed of three layers -- an outer impervious layer 222, an inner pervious liner 223, and an absorbent batt 220. The absorbent batt 220 is located well inside the elastic 230 at the back and also elastics 232 and 233 at the front. The diaper is shaped and has leg cutouts 212 and 214 with leg elastics 218 and 216 that are located within the leg portion of the diaper. In this embodiment, the front elastic is divided into two portions -- 232 and 233 -- having effective lengths between 240 and 241 and 238 and 239 respectively. A center portion 250 is not elasticized and would be located in the navel area of the infant. This open area may be any suitable size, but would be a minimum of about 1 inch and generally between 1 inch and about 2 inches. This embodiment 210 is illustrated also generally in the wearing position in FIG. 6 where the elasticized portions 232 and 233 form a separate elasticized front waist with the open portion 250 that is located where

-11-

the navel of the baby would be placed. It would also be within the invention to provide a divided elastic in the back. In certain manufacturing processes it may be easier to form the elastic in the front and back of identical construction.

The elastic film member forming the elastic waist in the back and front may be any of several known elastic materials. Among suitable materials is natural rubber. It is also possible to use heat-shrinkable materials such as those disclosed in U.S. Patent 3,912,565 to Koch et al. Other elastics which are suitable are those elastic adhesives such as disclosed in Bunnelle et al. U.S. Patent 4,259,220. The particular elastic material is not considered a part of this invention and the selection of particular elastic will be based on ease of application in manufacture and cost considerations. The elastic for the legs also may be any conventional material. The elastic may be any desired width. It is known that wider elastics are less likely to leave marks as the pressure is spread over a wider area.

The impervious layer may be any of the film materials that are well known in the art. A preferred material is polyethylene as it is flexible, of sufficient strength, and low in cost.

The absorbent batt material may be any of several absorbent materials. Included are materials such as fluff, which is divellicated fiber pulp. Also suitable are materials that are combinations of microfilaments of polypropylene and cellulose fibers such as disclosed in U.S. Patent 4,100,324 to Anderson et al.

The pervious liner material may be selected from any of the well-known materials such as spunbonded polymer film or

-12-

perforated polymer sheet materials. The preferred material is a spunbonded polypropylene web as this is pervious, comfortable, and low in cost.

The invention is suitable for both diapers that have a fastening tape that is usable only one time, and for those that are refastenable by means such as disclosed in U.S. 3,616,114 - Hamaguchi et al., and 3,951,149 - Ness et al.

The diaper of the invention has numerous advantages including better utilization of the absorbent batt prior to leaking. It further may be fastened more efficiently as the back is not elasticized to such a great extent that the tabs are hidden. The product is safe in that the amount of elastic is not such that the baby is likely to be closely held and the skin reddened or abraded. The limited width of elastic in the front further prevents rollover of the ends of the front that are overlapped by the wrapping of the back portion which can expose the fastening tape and damage the skin of the baby. The end seal of the diaper which is the portion where the pervious liner and the impervious outer cover are fastened together is improved by the elastic portion in the end.

CLAIMS

1. A disposable diaper comprising: a first outer layer in the form of a moisture impervious backing; an absorbent batt positioned in superposed relationship with respect to said backing, said batt being smaller than said backing and spaced inwardly from the sides and ends thereof; a second outer layer in the form of a moisture pervious facing positioned in superposed relationship with respect to said batt, said facing being larger than said batt and having marginal portions thereof secured to said backing; elastic means adapted to form leg elastics in each side portion of the diaper, and elastic means parallel to the margin of batt in the midportion of the ends of said diaper adapted to form an elastic waist, wherein the front end elastic means has an effective length located between about the center one-third and about two-thirds of the width of said impervious backing and wherein there is substantially no interaction between leg elastics and waist elastics.

2. The diaper of Claim 1 wherein the total waist elastic travel of said diaper in use is between about 1 inch and about 7 inches.

3. The diaper of Claim 1 wherein said waist elastic travel of said diaper in use is between about 2 inches and about 4 inches.

4. The diaper of Claim 1 wherein the effective length of the back elastic is located in an area of the diaper end between about the center one-third of the width and the inside ends of the diaper tape anchoring area.

-14-

5.  The diaper of Claim 1 wherein the end elastics are spaced at least one-fourth inch outwardly from the marginal edge of said batt.

6.  The diaper of Claim 1 wherein at least said front elastic is divided into two sections with a nonelastic portion in the middle area of the front end of the diaper where the wearer's navel would be located in use.

7.  The diaper of Claim 4 wherein the elastic at the diaper ends has a tension of between about 125 grams and about 175 grams.

8.  The diaper of Claim 1 wherein said diaper has an hour-glass shape forming a reduced width crotch portion.

9.  A disposable diaper comprising: a first outer layer in the form of a moisture impervious backing; an absorbent batt positioned in superposed relationship with respect to said backing, said batt being smaller than said backing and spaced inwardly from the sides and ends thereof; a second outer layer in the form of a moisture pervious facing positioned in superposed relationship with respect to said batt, said facing being larger than said batt and having marginal portions thereof secured to said backing; leg elastic means adapted to form leg elastics in each side portion of the diaper, and at least one waist elastic means in the midportion of at least one end of said diaper parallel to the marginal edge of said batt and adapted to form an elastic waist, wherein said at least one waist elastic means has an effective length of between about one-third and about two-thirds of the width of said impervious backing wherein there is substantially no

-15-

interaction between said leg elastics and said at least one waist elastic and wherein said at least one waist elastic has a tension of between about 100 and about 200 grams.

10. The diaper of Claim 9 wherein said at least one elastic comprises a single front elastic.

11. The diaper of Claim 9 wherein said at least one elastic comprises a single back elastic.

12. The diaper of Claim 9 wherein the waist elastic travel of said diaper in use is between about 2 inches and about 4 inches.

13. The diaper of Claim 9 wherein said tension is between about 125 and about 175 grams.

14. The diaper of Claim 9 wherein said at least one waist elastic is formed of two separate elastic pieces with a centered space between them.

15. A disposable elastic waist diaper wherein said waist elastic comprises front elastic adhered to between about one-third and two-thirds of the center of the end of the front of said diaper, the tension of said waist elastic is between about 60 and about 300 grams and the travel of said waist elastic is between about one and about seven inches.

16. The elastic waist diaper of Claim 15 wherein said waist elastic further comprises a back elastic extending between about one-third and six-tenths of the total width of said diaper.

17. The elastic waist diaper of Claim 15 wherein said travel is between about two inches and about four inches.

18. The elastic waist diaper of Claim 15 wherein said tension is between about 125 grams and about 175 grams.

19. The elastic waist diaper of Claim 15 wherein the said waist elastic is located between the edge of the diaper and a line parallel to and about one-eighth inch from the edge of the fluff.

0155636

FIG. 1

FIG. 2

FIG. 3

FIG. 6

0155636

FIG. 4

FIG. 5